(19) Europäisches Patentamt
European Patent Office
Office européen des brevets

(11) **EP 4 314 781 B1**

(12) **EUROPEAN PATENT SPECIFICATION**

(45) Date of publication and mention
of the grant of the patent:
**06.05.2026 Bulletin 2026/19**

(21) Application number: **22720308.0**

(22) Date of filing: **23.03.2022**

(51) International Patent Classification (IPC):
**G01N 21/71** *(2006.01)*     **G01J 3/443** *(2006.01)*
**G01J 3/44** *(2006.01)*      **G01N 33/204** *(2019.01)*

(52) Cooperative Patent Classification (CPC):
**G01N 21/718; G01J 3/44; G01J 3/443;
G01N 33/204**

(86) International application number:
**PCT/EP2022/057613**

(87) International publication number:
**WO 2022/200417 (29.09.2022 Gazette 2022/39)**

(54) **METHOD OF ADJUSTING THE ELEMENTAL INHOMOGENEITY OF A CONTINUOUS CAST METAL**

VERFAHREN ZUM ANPASSEN DER ELEMENTAREN INHOMOGENITÄT EINES STRANGGEGOSSENEN METALLS

PROCÉDÉ DE ADAPTER DE L'INHOMOGÉNÉITÉ ÉLÉMENTAIRE D'UN MÉTAL COULÉ EN CONTINU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.03.2021 EP 21164212**

(43) Date of publication of application:
**07.02.2024 Bulletin 2024/06**

(73) Proprietor: **Tata Steel Nederland Technology B.V.
1951 JZ Velsen-Noord (NL)**

(72) Inventors:
• **VAN DEN BERG, Frenk**
  **1970 CA IJMUIDEN (NL)**
• **SANTILLANA, Begona**
  **1970 CA IJMUIDEN (NL)**
• **HECHU, Kateryna**
  **1970 CA IJMUIDEN (NL)**

(74) Representative: **Group Intellectual Property
Services
c/o Tata Steel Nederland Technology B.V.
P.O. Box 10000 - 3G.37
1970 CA IJmuiden (NL)**

(56) References cited:
• **MEINHARDT CHRISTOPH ET AL: "Laser-induced
breakdown spectroscopy of scaled steel
samples taken from continuous casting
blooms", SPECTROCHIMICA ACTA. PART B:
ATOMIC SPECTROSCOPY, NEW YORK, NY, US,
US, vol. 123, 14 August 2016 (2016-08-14), pages
171 - 178, XP029721695, ISSN: 0584-8547, DOI:
10.1016/J.SAB.2016.08.013**
• **MIER D ET AL: "A stochastic model of the
process of sequence casting of steel, taking into
account imperfect mixing", APPLIED PHYSICS
B: LASERS AND OPTICS, SPRINGER
INTERNATIONAL, BERLIN, DE, vol. 125, no. 4, 1
April 2019 (2019-04-01), pages 1 - 7,
XP036753130, ISSN: 0946-2171, [retrieved on
20190401], DOI: 10.1007/S00340-019-7175-2**

- **ARAGON C ET AL: "IMPROVEMENTS IN QUANTITATIVE ANALYSIS OF STEEL COMPOSITION BY LASER-INDUCED BREAKDOWN SPECTROSCOPY AT ATMOSPHERIC PRESSURE USING AN INFRARED ND: YAG LASER", APPLIED SPECTROSCOPY, THE SOCIETY FOR APPLIED SPECTROSCOPY. BALTIMORE, US, vol. 53, no. 10, 1 October 1999 (1999-10-01), pages 1259 - 1267, XP000903123, ISSN: 0003-7028, DOI: 10.1366/0003702991945506**

**Description**

The present invention relates to a method of adjusting the elemental inhomogeneity of a continuously cast metal by laser-induced breakdown spectroscopy.

Continuous casting, also called strand casting, is the known process whereby molten metal, e.g. steel, is solidified into a billet, bloom, or slab. These billets blooms or slabs can be used for subsequent rolling in the finishing mills into wire, sections, plate or strip.

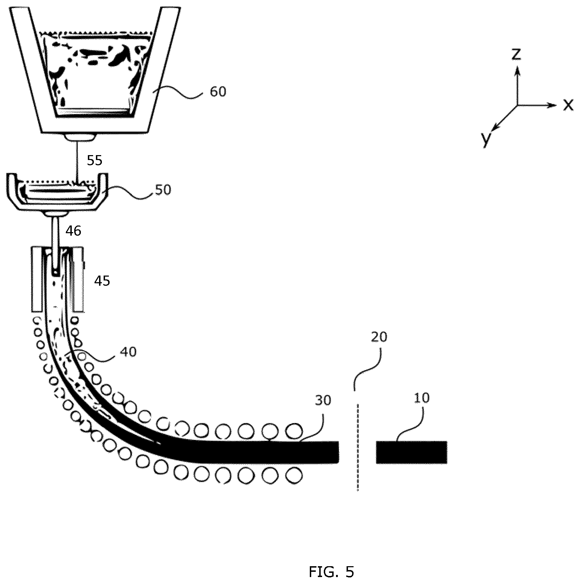

The molten metal is tapped into a ladle (Figure 5, 60) and after undergoing any ladle treatments, such as alloying and degassing, and arriving at the correct temperature, the ladle is transported to the top of a casting machine. Usually the ladle sits in a slot on a rotating turret at the casting machine. One ladle is in the 'on-cast' position (feeding the casting machine) while the other is made ready in the 'off-cast' position, and is switched to the casting position when the first ladle is empty.

From the ladle, the hot metal is transferred via a refractory pipe (55) to a holding bath called a tundish (50). The tundish allows a reservoir of metal to feed the casting machine while ladles are switched, thus acting as a buffer of hot metal, as well as smoothing out flow, regulating metal feed to the moulds and cleaning the metal (figure 5).

Metal is drained from the tundish through another shroud into the top of an open-base copper mould (45). The depth of the mould can range from 0.5 to 2 metres depending on the casting speed and section size. The mould is water-cooled to solidify the hot metal directly in contact with it; this is the primary cooling process. It also oscillates to prevent the metal sticking to the mould walls. A lubricant is added to the metal in the mould to prevent sticking, and to trap any slag particles-including oxide particles or scale-that may be present in the metal and bring them to the top of the pool to form a floating layer of slag. The shroud is set so the hot metal exits it below the surface of the slag layer in the mould and is thus called a submerged entry nozzle (SEN).

In the mould, a thin shell of metal next to the mould walls solidifies before the middle section, now called a strand (40), exits the base of the mould into a spray chamber. The bulk of metal within the walls of the strand is still molten. The strand is immediately supported by closely spaced, water-cooled rollers which support the walls of the strand against the ferrostatic pressure of the still-solidifying liquid within the strand. To increase the rate of solidification, the strand is sprayed with large amounts of water as it passes through the spray-chamber; this is the secondary cooling process. Final solidification of the strand may take place after the strand has exited the spray-chamber.

After exiting the spray-chamber, the strand (30) passes through straightening rolls and withdrawal rolls. Finally, the strand is cut into predetermined lengths by mechanical shears or by travelling oxyacetylene torches (20), is marked for identification, and is taken either to a stockpile or to the next forming process.

Inhomogeneity of a cast metal may have detrimental effects on the final properties of the product, for example, due to macrosegregation. In the steel industry, for instance, macrosegregation in a steel cast product may be detrimental to the properties of the final product. Steels with elemental inhomogeneity will be less suitable for welded tube due to worse joining performance as well as for beam blanks used in rail products and safety parts of the automotive chassis due to the lack of structural integrity. For product development, process control and quality assurance it is desired to provide an early indication of the inhomogeneity of the cast steel. However, with the currently available techniques, the assessment takes too much manpower, time and money for inspection of all slabs produced.

From the article "A stochastic model of the process of sequence casting of steel, taking into account imperfect mixing" by D. Mier et al. in Applied Physics B: Lasers and Optics, vol.125:65, no.4, 01 April 2019 it is known to compare a numerical model of cast steel inhomogeneity with laser-induced breakdown spectroscopy measurements on a polished cold billet.

It is therefore desirable to provide a method that can swiftly give insight in the elemental inhomogeneity of the cast metal.

It is therefore an object of the invention to provide a method that rapidly assesses the inhomogeneity, preferably within 3 - 180 minutes or faster.

It is also an object of the invention to provide a method that limits the preparation time of the metal sample.

It is also an object of the invention to provide a method that can measure cast metals at elevated temperatures.

It is also an object of the invention to provide a method that can be used online during metal casting.

One or more of these objectives are reached with a method according to claim 1 - 15.

In the first aspect, the invention provides a method according to claim 1.

According to the invention, the cross-section of the continuously cast metal is assessed by LIBS in a plurality of spots, such that the deviation from the aimed elemental composition can be determined at each spot. LIBS allows for the simultaneous analysis of several common elements present in a metal slab such as Mn, Si, Al, Cr, C, O, P, S, Ti, V, Ni, Mo. By comparing the elemental composition in a plurality of spots compared to the aimed elemental composition, the inhomogeneity of the cross-section can be determined rapidly. As inhomogeneity of the cast metal may have detrimental effects on the final properties of the metal a swift and reliable analysis of the elemental composition after casting can be used to classify the metal into different categories or even reject the cast metal, thereby ensuring that the cast metal as-is is suitable for the processes and applications further down the line, thus minimizing post-process rejections.

The plane of the cross-section of the continuously cast metal is not particularly limited and can be taken in any direction where inhomogeneity is expected. The cross section may be longitudinal (parallel to the casting direction of the metal slab) or transverse (perpendicular to the casting direction of the metal slab). Preferably the cross-section is a transverse cross-section.

Preferably, the LIBS measurement is carried out under a protected atmosphere, to reduce the oxidation rate and to increase the number density of atoms in the excited state in the plasma. Preferably, at least the area of the cross-section to be measured is under a protected atmosphere. The protected atmosphere may include noble gasses such as neon (Ne), argon (Ar), krypton (Kr) or xenon (Xe). Preferably, argon (Ar) is used, for example by blowing a stream of argon on the surface.

The method of the invention can be used for any type of cast metal, such as slabs, billets and blooms.

In an embodiment of the invention, the laser has a laser pulse energy of at least 10 mJ per pulse, preferably at least 22 mJ per pulse. Generally, the as-cast slab may suffer from an oxide layer on the cross-section. By increasing the laser power, the inventors found that the oxide layer is penetrated by the laser, and the segregation of for example Mn can still be reliably measured. Without wishing to be bound to theory, it is believed that this oxide layer is penetrated by the laser through drilling, melting and evaporation of the oxide. This eliminates the need for descaling the sample before measuring. The maximum laser power is not particularly limited but is preferably at most 150 mJ to reduce splashes of molten metal around the crater.

In an embodiment of the invention, the elemental inhomogenity of the whole area of the cross-section of the cast metal is determined. The whole area is scanned spot-wise. The results can then be analysed to provide an indication of the inhomogeneity, for example by plotting the results for each chemical element for each spot in the cross-section in a colour-coded plot.

In an embodiment of the invention, the step size is preferably in a range of 20 - 500 $\mu$m, in order to obtain a good resolution in a short time frame. A step size below 20 $\mu$m will take more time and will not provide valuable additional information. Preferably the step size is at least 20 $\mu$m, more preferably at least 30 $\mu$m, most preferably at least 50 $\mu$m. A step size above 500 $\mu$m will result in a low resolution, and a potential underestimation of the amount of inhomogeneity in the metal, therefore the step size is preferably at most 500 $\mu$m, more preferably at most 300 $\mu$m, most preferably at most 200 $\mu$m.

In general, the size of the cross-section is not particularly limited and can be in a range from a small region of a four-spot measurement to the full size of the cross-section of the cast metal, typically 225 x 2200 mm for a metal slab in a transverse section, scanning thousands of spots. In an embodiment of the invention, the area to be measured is in a range of 0.01 - 10 % of the overall cross-sectional area of the cast metal, more preferably 0.05 - 5 % of the overall cross-sectional area, most preferably 0.10 - 1 % of the overall cross-sectional area. By analysing a part of the cross-section, time and energy for measurement is reduced, while a clear indication of the inhomogeneity can still be obtained. Reducing measurement time is particularly important for online measurements. The area to be measured will be chosen based on the specification and expected inhomogeneity of the cast metal, e.g. based on solidification profile of the caster and type of product to be cast (billet, bloom, slab).

In an embodiment of the invention, each spot is agitated at least three times. It was found by the inventors that three laser shots per spot resulted in a reliable signal while reducing the required processing time. Preferably each spot is agitated at most fifteen times, to minimize the ablation around the crater and keep the scan time in reasonable limits.

In an embodiment of the invention, each spot has a crater diameter after agitation in a range of 20 - 500 $\mu$m. In order to increase the likelihood to detect the macrosegregation islands in the steel, the crater diameter is preferably at least 20 $\mu$m, more preferably at least 30 $\mu$m, most preferably at least 50 $\mu$m. The crater diameter is preferably at most 500 $\mu$m, more preferably at most 300 $\mu$m, most preferably at most 200 $\mu$m, to ensure a good resolution of the measurement, as a crater diameter which is much larger than the segregation islands, results in a smeared-out signal.

In an embodiment of the invention, the laser spot diameter d, corresponding to the focused laser diameter at the cross-section, is in the range of 100 - 300 $\mu$m, in order to provide an optimum between position resolution and measurement speed. Below 100 $\mu$m the analysis requires more processing time. Above 300 $\mu$m the resolution becomes low, as the spot becomes too coarse for segregation islands, being typically in the order of 10 - 50 $\mu$m. It was observed by the inventors that a measurement spot, which is much larger than the island, results in a smeared-out signal and a smoothed map with lower resolution.

In an embodiment of the invention the energy density per spot is at least 60 mJ/mm$^2$, the energy density being determined with the following formula:

$$\frac{n * p}{\pi * (d/2)^2}$$

wherein n corresponds with the number of agitations per spot, p corresponds to the laser pulse energy and d corresponds to the laser spot diameter. An energy density per spot of at least 60 mJ/mm$^2$ ensures sufficient penetration of the metal surface, ensuring a good signal-noise ratio per spot.

In an embodiment of the invention the area analysis rate is at least 10 $\mu$m$^2$/s, preferably at least 100 $\mu$m$^2$/s, the area analysis rate being determined with the following formula:

$$\frac{\pi * (d/2)^2 * f}{n}$$

wherein d corresponds to the laser spot diameter, f to the laser frequency and n to the number of agitations per spot. With an area analysis rate of at least 10 $\mu m^2/s$ a swift measurement of the area is possible, thereby allowing the analysis to be used in time-pressing situations, such as an online measurement.

In an embodiment of the invention, the cross-section may be prepared before laser agitation, by grinding or drilling or remachining or deburring or a combination thereof. Depending on the metal cast to be measured, a preparation of the surface of the cross-section may be desirable. For example, if the metal slab has an oxide scale of more than 20 $\mu m$ or if the surface of the cross-section is distorted due to processing steps such as slicing.

In an embodiment of the invention, the elemental inhomogeneity of the cast metal is determined offline. For an offline measurement, a sample of the continuously cast metal can be taken and analysed with LIBS. This has the advantage that additional time is available for the scan, which may be used to obtain a higher resolution image of the inhomogeneity of the sample. This can be realized for example by minimizing the step size and the laser size.

According to the invention, the elemental inhomogeneity of the cast metal is determined online. An online measurement provides immediate feedback about the inhomogeneity of the cast metal, and thereby allows for adjustments in the process, both backward (casting parameter setting) and forward (process conditions and/or and/or rescheduling and/or perspective product quality). For this embodiment, the sample should be measured in a relatively short time. Depending on the casting speed of the metal, and thus the available processing time, the measurement time has to be adjusted, preferably within 1 - 60 minutes, to keep up the measurement speed with the casting speed, such that the overall steelmaking process is not delayed. Therefore, in online measurements, the resolution of the scan can be lowered. This can be realized by maximizing the step size and/or laser diameter and/or by increasing laser power and/or laser frequency. The online measurement can be performed directly on the cast metal. Alternatively, the measurement can be performed on a probe taken from the cast metal.

In an embodiment of the invention, the continuously cast metal has a temperature in a range of room temperature - 1000 °C. 1000 °C is the maximum temperature of the cast metal upon exit of the caster, and the offline measurements may be carried out at environmental (or room) temperature. Advantageously, the method of the invention works in a large temperature range, including high temperatures in a range of 700 - 1000 °C, such that the metal cast does not require cooling down, before analysing online.

In an embodiment of the invention, the information obtained by LIBS is analysed and presented on a human-machine interface. The information as obtained by LIBS may be presented on a human-machine interface as a colour-coded map showing the inhomogeneity of the sample or as a simple message showing that the cast metal is inside or outside a predetermined specification related to the inhomogeneity.

In an embodiment of the invention, the casting parameters are changed based on the feedback of the LIBS to improve product quality. Typical casting parameters that can be adjusted to change macrosegregation are primary and secondary cooling profiles, fluid flow, casting speed, superheat, mechanical changes such as soft reduction (SR) and dynamic soft reduction (DSR) and stirring practises such as EMS (ElectroMagnetic Stirring) and EMBr (ElectroMagnetic Brake).

In a preferred embodiment of the invention, the metal is steel.

Thus, the invention provides a method of adjusting the elemental inhomogeneity of a continuously cast metal according to claim 1.

Examples of casting parameters that can be adjusted to change macrosegregation are typically primary and secondary cooling profiles, fluid flow, casting speed, superheat, mechanical changes such as soft reduction and dynamic soft reduction (SR and DSR) and stirring practises (EMS and EMBr).

The invention is further explained by the non-limiting examples shown in FIG. 1 - 5.

FIG. 1 shows the principles of LIBS.
FIG. 2 shows the effect of laser pulse energy and number of agitations on the cross section.
FIG. 3 shows LIBS results obtained by a method according to the invention.
FIG. 4 shows an online LIBS measurement setup.
FIG. 5 shows a known continuous casting setup.

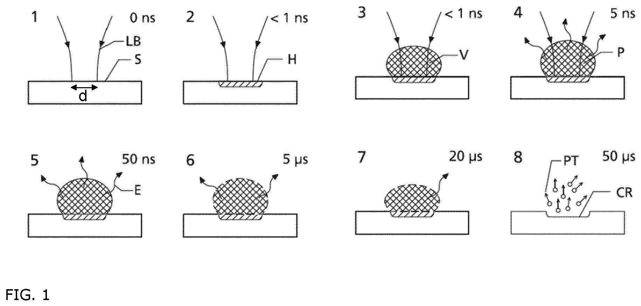

In FIG. 1 the principle of laser-induced breakdown spectroscopy is illustrated schematically. A pulsed laser beam is focused at a spot on the cross-section of the cast metal, with a laser spot diameter d. The radiation energy is deposited on the spot of the cross-section (2) and then the material evaporates (3) and a plasma is generated (4) within the material vapour and the surrounding argon atmosphere. The plasma emits radiation with wavelengths which vary for the specific elements. The radiation is detected by a spectrometer for each agitation to provide a value for the elements of interest. The evaporated material is partially removed from the surface, which leads to the formation of a crater (8).

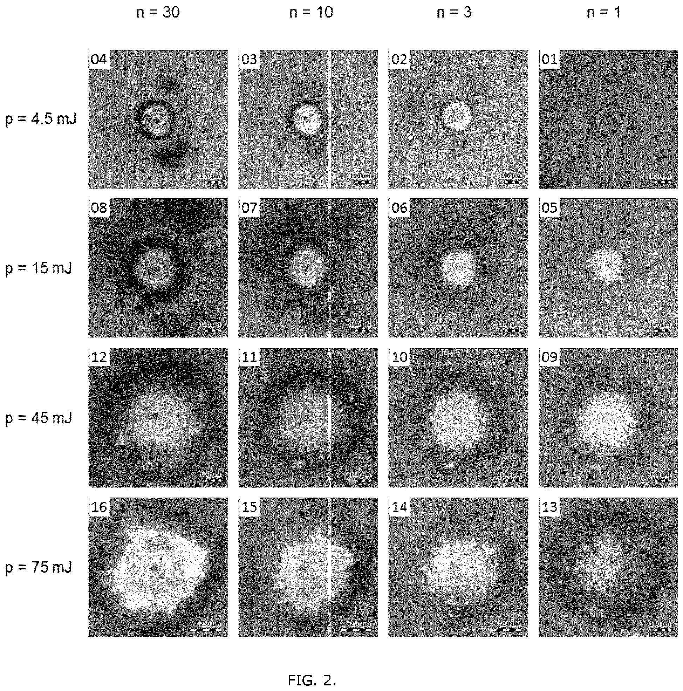

FIG. 2. shows confocal images of 16 spots in a cross section of a cast steel after LIBS analysis at different laser pulse energy (p) and number of agitations (n) to determine the optimal parameters for the LIBS analysis. The bar in samples 01 - 13 corresponds to 100 $\mu$m, whereas the bar in samples 14 - 16 corresponds to 250 $\mu$m. A high laser pulse energy (13 - 16) resulted in a large crater size (>500 $\mu$m) as well as splashes and was therefore less suitable for LIBS analysis of the microsegregation. A low laser pulse energy (01 - 04) resulted in a crater size of about 100 $\mu$m and provided a weak signal, especially at a low number of agitations. At a low number of agitations the penetration depth was much lower, whereas at a high number of agitations the scan time required would be increased. The inventors found that spots 06, 07, 10 and 11 represented the best settings to ensure no splashes, sufficient penetration, good signal and a swift analysis time.

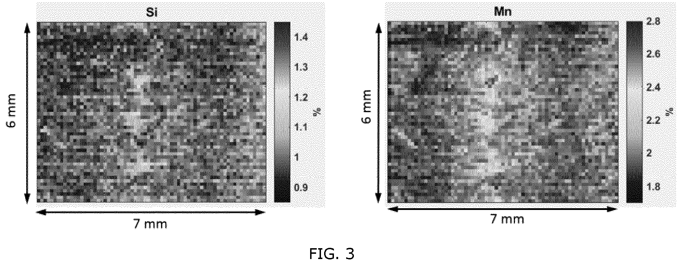

FIG. 3 shows an elemental composition colour-coded map for a cast steel with the optimal settings as determined from the confocal analysis. The composition of the cast steel in weight percentage is 1% Si, 2% Mn, 0.22% C, rest Fe. 0.6% of the cross-sectional surface of the cast steel is analysed with LIBS. Each spot on the cross-section is agitated 5 times, at an energy of 22 mJ in order to ensure a good signal to noise ratio. The radiation is detected by a spectrometer at 288.1 nm, 403.1 nm and 438.5 nm for each agitation to provide a value for Mn, Si and Fe, respectively. For each spot, the signal of the alloys is normalized by Fe. With a step size of 100 $\mu$m an area of 6 by 7 mm is scanned which leads to a colour-coded map of the respective area of that transverse section for Mn (FIG. 2A) and Si (FIG. 2B), clearly showing inhomogeneity in the sample.

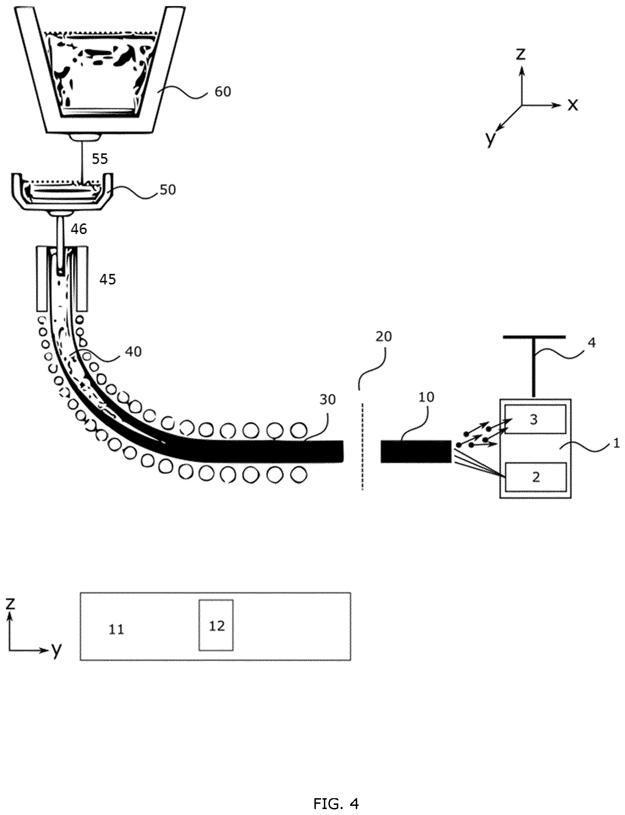

FIG. 4 shows an embodiment of the method of the invention in an online setting at a continuous casting installation. Molten metal is tapped into a ladle 60. In the ladle, the molten metal may undergo treatments such as alloying and degassing to prepare the desired chemical composition. The molten metal is transferred to a tundish (50) and during casting the strand of molten metal (30, 40) is solidified. After complete solidification (at the "metallurgical length" of the caster) the strand is cut (20), for example with oxyacetylene torches into a slab (10). At the cut, the inhomogeneity of the cross-sectional surface is determined with a LIBS unit (1), which can move in the x, y and z direction. One or more spots in the desired region (12) of the area of the cross-section (11) are agitated by a laser (2) and the resulting plasma is analysed with a spectrometer (3) to determine the inhomogeneity of each spot. Depending on the speed of the strand, the LIBS unit may travel in the same x direction and at the same speed as the slab. Several slabs may be measured accordingly. The cross-sectional surface to be measured may be optionally prepared with an online grinder (not shown). A heat map or a measure of inhomogeneity will be placed on a human machine interface as an input for the operators upstream or downstream.

FIG. 5 shows a known continuous casting setup as described in the introduction part of the description.

## Claims

1. A method of adjusting the elemental inhomogeneity of a continuously cast metal, comprising the steps of casting a metal through a mould with one or more casting parameters, determining the elemental inhomogeneity of the solidified cast metal, thereby determining if the metal cast is homogeneous or inhomogeneous, and maintaining the casting parameters to the present state when it is determined that the metal cast is homogeneous and adjusting one or more casting parameters when it is determined that the metal cast is inhomogeneous, thereby adjusting the metal cast to be more homogeneous, wherein the elemental inhomogeneity of the continuously cast metal is determined online by laser-induced breakdown spectroscopy, comprising the steps of providing a cross-section of the continuously cast metal, agitating a plurality of spots within the cross-section with a laser thereby generating a plasma and analysing the radiation emitted by the plasma of each spot to determine the elemental composition of each spot and deduce the deviation from the aimed elemental composition, and wherein the one or more casting parameters comprise primary and secondary cooling profiles, fluid flow, casting speed, superheat, mechanical changes such as soft reduction (SR) and dynamic soft reduction (DSR) and stirring practises (EMS and EMBr).

2. The method according to claim 1, wherein the laser has a laser pulse energy of at least 10 mJ per pulse, preferably at least 22 mJ per pulse.

3. The method according to any of the claims 1 or 2, wherein the elemental inhomogeneity of the whole area of the cross-section is determined.

4. The method according to any of the claims 1 or 2, wherein the elemental inhomogeneity of 0.01 - 10 % of the cross-section is determined.

5. The method according to any of the preceding claims, wherein the step size between the spots is in the range of 20 - 500 $\mu$m.

6. The method according to any of the preceding claims, wherein each spot is agitated at least 3 times.

7. The method according to any of the preceding claims, wherein the laser spot diameter is in the range of 100 - 300 $\mu$m.

8. The method according to any of the preceding claims, wherein the energy density per spot is at least 60 mJ/mm$^2$, the energy density being determined with the following formula:

$$\frac{n * p}{\pi * (d/2)^2}$$

wherein n corresponds with the number of agitations per spot, p corresponds to the laser pulse energy and d corresponds to the laser spot diameter.

9. The method according to any of the preceding claims, wherein the area analysis rate is at least 10 $\mu m^2$/s, preferably at least 100 $\mu m^2$/s, the area analysis rate being determined with the following formula:

$$\frac{\pi * (d/2)^2 * f}{n}$$

wherein d corresponds to the laser spot diameter, f to the laser frequency and n to the number of agitations per spot.

10. The method according to any of the preceding claims, wherein the cross-section is prepared before laser agitation, by grinding or drilling or remachining or deburring or a combination thereof.

11. The method according to any of the claims above, wherein the continuously cast metal has a temperature in a range of room temperature - 1000 °C.

12. The method according to any of the preceding claims, wherein the information obtained by LIBS is analysed and presented on a human-machine interface.

13. The method according to any of the preceding claims, wherein the cross section is transverse, i.e. perpendicular to the casting direction of the metal slab.

14. The method according to any of the preceding claims, wherein the elemental inhomogeneity of the continuously cast metal is determined in a temperature range of 700 - 1000 °C.

15. The method according to any of the claims above, wherein the metal is steel.

**Patentansprüche**

1. Verfahren zum Anpassen der elementaren Inhomogenität eines stranggegossenen Metalls, umfassend die Schritte des Gießens eines Metalls durch eine Form mit einem oder mehreren Gussparametern, des Bestimmens der elementaren Inhomogenität des erstarrten gegossenen Metalls, wodurch bestimmt wird, ob der Metallguss homogen oder inhomogen ist, und des Beibehaltens der Gussparameter auf dem gegenwärtigen Zustand, wenn bestimmt wird, dass der Metallguss homogen ist, und des Anpassens eines oder mehrerer Gussparameter, wenn bestimmt wird, dass der Metallguss inhomogen ist, wodurch der Metallguss angepasst wird, um homogener zu werden, wobei die elementare Inhomogenität des stranggegossenen Metalls online durch laserinduzierte Durchbruchspektroskopie bestimmt wird, umfassend die Schritte des Bereitstellens eines Querschnitts des stranggegossenen Metalls, des Hin- und Herbewegens einer Vielzahl von Punkten innerhalb des Querschnitts mit einem Laser, wodurch ein Plasma erzeugt wird, und des Analysierens der vom Plasma von jedem Punkt emittierten Strahlung, um die elementare Zusammensetzung von jedem Punkt zu bestimmen und die Abweichung von der angestrebten elementaren Zusammensetzung abzuleiten, und wobei der eine oder die mehreren Gussparameter primäre und sekundäre Kühlprofile, Fluidfluss, Gießgeschwindigkeit, Überhitzung, mechanische Änderungen, wie Soft Reduction (SR) und Dynamic Soft Reduction (DSR) sowie Rührpraktiken (EMS und EMBr), umfassen.

2. Verfahren nach Anspruch 1, wobei der Laser eine Laserpulsenergie von mindestens 10 mJ pro Puls, vorzugsweise mindestens 22 mJ pro Puls, aufweist.

3. Verfahren nach einem der Ansprüche 1 oder 2, wobei die elementare Inhomogenität des gesamten Bereichs des Querschnitts bestimmt wird.

4. Verfahren nach einem der Ansprüche 1 oder 2, wobei die elementare Inhomogenität von 0,01 - 10 % des Querschnittes bestimmt wird.

5. Verfahren nach einem der vorhergehenden Ansprüche, wobei die Schrittgröße zwischen den Punkten im Bereich von 20 - 500 $\mu m$ liegt.

6. Verfahren nach einem der vorhergehenden Ansprüche, wobei jeder Punkt mindestens dreimal hin- und herbewegt wird.

7. Verfahren nach einem der vorhergehenden Ansprüche, wobei der Laserpunktdurchmesser im Bereich von 100 - 300 $\mu m$ liegt.

8. Verfahren nach einem der vorhergehenden Ansprüche, wobei die Energiedichte pro Punkt mindestens 60 mJ/mm$^2$ beträgt, wobei die Energiedichte mit der folgenden Formel bestimmt wird: $\frac{n * p}{\pi * (d/2)^2}$ ,

wobei n der Anzahl an Hin- und Herbewegungen pro Punkt entspricht, p der Laserpulsenergie entspricht und d dem Laserpunktdurchmesser entspricht.

9. Verfahren nach einem der vorhergehenden Ansprüche, wobei die Flächenanalyserate mindestens 10 $\mu m^2$/s, vorzugsweise mindestens 100 $\mu m^2$/s beträgt, wobei die Flächenanalyserate mit der folgenden Formel bestimmt wird: $\dfrac{\pi * (d/2)^2 * f}{n}$ , wobei d dem Laserpunktdurchmesser, f der Laserfrequenz und n der Anzahl an Hin- und Herbewegungen pro Punkt entspricht.

10. Verfahren nach einem der vorhergehenden Ansprüche, wobei der Querschnitt vor Laser-Hin- und Herbewegung durch Schleifen oder Bohren oder Nachbearbeiten oder Entgraten oder eine Kombination davon hergestellt wird.

11. Verfahren nach einem der oben genannten Ansprüche, wobei das stranggegossene Metall eine Temperatur in einem Bereich von Raumtemperatur - 1000 °C aufweist.

12. Verfahren nach einem der vorhergehenden Ansprüche, wobei die durch LIBS erhaltenen Informationen auf einer Mensch-Maschine-Schnittstelle analysiert und präsentiert werden.

13. Verfahren nach einem der vorhergehenden Ansprüche, wobei der Querschnitt quer, d. h. senkrecht zur Gießrichtung der Metallbramme, ist.

14. Verfahren nach einem der vorhergehenden Ansprüche, wobei die elementare Inhomogenität des stranggegossenen Metalls in einem Temperaturbereich von 700 - 1000 °C bestimmt wird.

15. Verfahren nach einem der oben genannten Ansprüche, wobei das Metall Stahl ist.

**Revendications**

1. Procédé d'adaptation de l'inhomogénéité élémentaire d'un métal coulé en continu, comprenant les étapes de coulée d'un métal à travers un moule avec un ou plusieurs paramètres de coulée, de détermination de l'inhomogénéité élémentaire du métal coulé solidifié, ce qui permet de déterminer si le métal coulé est homogène ou inhomogène, et de maintien des paramètres de coulée à l'état présent lorsqu'il est déterminé que le métal coulé est homogène et d'adaptation d'un ou plusieurs paramètres de coulée lorsqu'il est déterminé que le métal coulé est inhomogène, ce qui permet d'adapter le métal coulé pour qu'il soit plus homogène, dans lequel l'inhomogénéité élémentaire du métal coulé en continu est déterminée en ligne par spectroscopie par claquage induit par laser, comprenant les étapes de fourniture d'une section transversale du métal coulé en continu, d'agitation d'une pluralité de points dans la section transversale avec un laser générant ainsi un plasma et d'analyse du rayonnement émis par le plasma de chaque point pour déterminer la composition élémentaire de chaque point et en déduire l'écart par rapport à la composition élémentaire visée, et dans lequel lesdits un ou plusieurs paramètres de coulée comprennent les profils de refroidissement primaire et secondaire, le débit de fluide, la vitesse de coulée, la surchauffe, les changements mécaniques tels que la réduction douce (SR) et la réduction douce dynamique (DSR) et les pratiques d'agitation (EMS et EMBr).

2. Procédé selon la revendication 1, dans lequel le laser présente une énergie d'impulsion laser d'au moins 10 mJ par impulsion, de préférence d'au moins 22 mJ par impulsion.

3. Procédé selon l'une quelconque des revendications 1 ou 2, dans lequel l'inhomogénéité élémentaire de toute la surface de la section transversale est déterminée.

4. Procédé selon l'une quelconque des revendications 1 ou 2, dans lequel l'inhomogénéité élémentaire de 0,01 à 10 % de la section transversale est déterminée.

5. Procédé selon l'une quelconque des revendications précédentes, dans lequel la taille de pas entre les points est comprise dans la plage de 20 à 500 $\mu$m.

6. Procédé selon l'une quelconque des revendications précédentes, dans lequel chaque point est agité au moins 3 fois.

7. Procédé selon l'une quelconque des revendications précédentes, dans lequel le diamètre de point laser est compris dans la plage de 100 à 300 $\mu$m.

8. Procédé selon l'une quelconque des revendications précédentes, dans lequel la densité d'énergie par point est d'au moins 60 mJ/mm$^2$, la densité d'énergie étant déterminée à l'aide de la formule suivante :

$$\frac{n * p}{\pi * (d/2)^2}$$

dans laquelle n correspond au nombre d'agitations par point, p correspond à l'énergie d'impulsion laser et d correspond au diamètre de point laser.

**9.** Procédé selon l'une quelconque des revendications précédentes, dans laquelle la vitesse d'analyse surfacique est d'au moins 10 $\mu$m²/s, de préférence d'au moins 100 $\mu$m²/s, la vitesse d'analyse surfacique étant déterminée par la formule suivante :

$$\frac{\pi * (d/2)^2 * f}{n}$$

dans laquelle d correspond au diamètre de point laser, f à la fréquence laser et n au nombre d'agitations par point.

**10.** Procédé selon l'une quelconque des revendications précédentes, dans lequel la section transversale est préparée avant l'agitation au laser, par meulage ou perçage ou réusinage ou ébavurage ou une combinaison de ceux-ci.

**11.** Procédé selon l'une quelconque des revendications ci-dessus, dans laquelle le métal coulé en continu présente une température dans la plage de la température ambiante à 1000°C.

**12.** Procédé selon l'une quelconque des revendications précédentes, dans lequel les informations obtenues par LIBS sont analysées et présentées sur une interface homme-machine.

**13.** Procédé selon l'une quelconque des revendications précédentes, dans lequel la section transversale est transverse, c'est-à-dire perpendiculaire à la direction de coulée de la brame métallique.

**14.** Procédé selon l'une quelconque des revendications précédentes, dans lequel l'inhomogénéité élémentaire du métal coulé en continu est déterminée dans la plage de température de 700 à 1000°C.

**15.** Procédé selon l'une quelconque des revendications ci-dessus, dans lequel le métal est de l'acier.

FIG. 1

FIG. 2.

FIG. 3

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **D. MIER et al.** A stochastic model of the process of sequence casting of steel, taking into account imperfect mixing. *Applied Physics B: Lasers and Optics*, 01 April 2019, vol. 125 (4), 65